Europäisches Patentamt

European Patent Office

Office européen des brevets

(11) Publication number: **0 415 845 A1**

# EUROPEAN PATENT APPLICATION

(21) Application number: **90402381.9**

(51) Int. Cl.⁵: **A61L 27/00**

(22) Date of filing: **28.08.90**

(30) Priority: **28.08.89 JP 221056/89**

(43) Date of publication of application:
**06.03.91 Bulletin 91/10**

(84) Designated Contracting States:
**BE DE FR GB IT NL SE**

(71) Applicant: **TERUMO KABUSHIKI KAISHA
No. 44-1, Hatagaya 2-chome, Shibuya-ku
Tokyo 151(JP)**

(72) Inventor: **Ohsaki, Kenichi, c/o Terumo K.K.
1500, Inokuchi, Nakai-cho
Ashigarakami-gun, Kanagawa-ken(JP)**
Inventor: **Yamazaki, Motoshi, c/o Terumo K.K.
1500, Inokuchi, Nakai-cho
Ashigarakami-gun, Kanagawa-ken(JP)**

(74) Representative: **Gillard, Marie-Louise et al
Cabinet Beau de Loménie 55, Rue
d'Amsterdam
F-75008 Paris(FR)**

(54) Implant materials for long-term implantation and process for preparing the same.

(57) The invention relates to implants materials which can be implanted in vivo for a long term. The materials comprise a base material coated with water-soluble high-molecular substance wherein said high-molecular substance is combined with a reactive functional group(s) on the surface of the base material by an ionic or a covalent bond. The implant materials can be implanted for a long term subcutaneously, subepitherially, submucosally, intraperitoneally, in the thoracic cavity, intraviscerally or in the adipose tissue.

EP 0 415 845 A1

# IMPLANT MATERIALS FOR LONG-TERM IMPLANTATION AND PROCESS FOR PREPARING THE SAME

## Background of the Invention

### Field of the Invention

This invention relates to implant materials which can be implanted subcutaneously, subepitherially, submucosally, intraperitoneally, in the thoracic cavity, intraviscerally or in the adipose tissue over a long term. It is also concerned with a process for preparing the implant materials.

### Description of the Prior Art

Implant materials presently available include medical silicones, apatites, and synthetic high-molecular substances such as polyurethanes, polyesters, and polypropylene. For example, medical silicones are utilized for artificial breast, tissue expander or the like and applications of apatites to artificial bones and root of a tooth etc. are investigated.

These synthetic high-molecular substances except for the medical silicones are highly foreign materials to organisms, and cause inflammation when implanted in living organism over a long term. Thus, these substances have been problematic from the viewpoint of biocompatibility. On the other hand, in addition to being biocompatible, such materials for implantation are generally required to meet the following physico-engineering conditions:

(1) To have required physical properties such as strength,   ·

(2) to be easily fabricated or molded,

(3) to exhibit no change in chemical and physical properties during a long-term implantation in living organism, and

(4) to be sterilizable.

While medical silicones have a relatively improved biocompatibility and cause less inflammation during a long-term implantation, they have a tendency to peripherally form a thick capsule and an insufficient mechanical strength, which lead to their limited use in limited region in view of the above conditions. Apatites are defective in that they also have a limitation in region and method for use and difficulties in fabrication and molding due to occurrence of the capsule formation and lack of fexibility. The capsule formation induces bleeding, pain and contracture. It is also pointed out that the capsule formation is related to carcinogenesis.

## Summary of the Invention

It is an object of the invention to provide an implant material that are biocompatible with less tendency of forming capsule and meet the above-mentioned physico-engineering conditions with no limitation in region and method for use.

We have now found that the above-mentioned problem can be solved by forming on the surface of an in vivo artificial organ a hydrated layer comprising a water-soluble high-molecular substance via a reactive functional group.

Thus, the long-term implant materials according to the invention are characterized in that by ionically or covalently bonding a reactive functional group present on the implant material with a water-soluble high-molecular substance or its derivative, at least the surface of the material to be in contact with biotissues is coated with the water-soluble high-molecular substance or its derivatives.

Constitution of the invention will be described in particular below.

The implant material for long-term implantation herein means a member to be indwelt in the body continuously for a period of one month or longer, for example, subcutaneously, subepitherially, submucosally, intraperitoneally, in the thoracic cavity, intraviscerally or in the adipose tissue. The member includes

(1) Subcutaneously: Imbedded port for drug infusion, artificial breast, tissue expander, artificial testicle, artificial penis, artificial auricle, heart pace maker, artificial nose and artificial anus,

(2) Submucosally: Artificial root of a tooth,

(3) Intraperitoneally or in the thoracic cavity: Adhesion-preventive membrane, artificial heart and artificial blood vessel,

(4) Intraviscerally: Artificial bone, artificial joint, artificial tendon, artificial urethra, artificial blood vessel and artificial valve,

(5) In the adipose tissue: Artificial spleen, and

(6) Others: Artificial trachea, artificial esophagus and artificial pharynx.

In the plant materials of the invention, the artificial organ may be such that at least the surface of the artificial organ to be in contact with biotissues is coated with a water-soluble high-molecular substance or a derivative thereof. The water-soluble high-molecular substance used for forming the coat layer is fixed on the base material by an ionic bond or a covalent bond. Such water-soluble macromolecular substance is, in principle, in non-cross linked chain form and contains hydrophilic groups such as -OH, -CHNH$_2$, -COOH, -NH$_2$. -COO$^-$ -SO$_3^-$ or -NR$_3^+$, which includes natural water-soluble high-molecular substances such as those of

1) starch group including carboxymethyl starch and dialdehyde starch,

2) cellulose group including CMC, MC, HEC and HPC,

3) tannin and lignin group including tannin and lignin,

4) polysaccharide group including alginic acid, gum arabic, guar gum, tragacanth gum and tamarind species and

5) protein group including gelatin, casein, glue and collagen, and synthetic water-soluble high-molecular substances such as those of

1) PVA group including polyvinyl alcohol,

2) polyethylene oxide group including polyethylene oxide and polyethylene glycol,

3) acrylic acid group including sodium polyacrylate,

4) maleic anhydride group including methyl vinyl ether-maleic anhydride copolymer,

5) phthalic acid group including polyhydroxyethyl phthalate,

6) water-soluble polyester group including polydimethylol propionate,

7) ketone-aldehyde resin group including methyl isopropyl ketone-formaldehyde resin,

8) acrylamide group including polyacrylamide,

9) polyvinylpyrrolidone group including PVP,

10) polyamine group including polyethylenimine,

11) poly-electrolyte group including polystyrene sulfonate and

12) water-soluble polyamide group.

A derivative thereof as used herein may not necessarily be water soluble and include any of those which contain said water-soluble high-molecular substance as basic constituent. As described below, a derivative, even if insolubilized, may be used provided that its molecular chain has a degree of freedom and it can be hydrated.

The derivatives include, for example, esters, salts, amides, anhydrides, halides, ethers, hydrolyzates, acetals, formals, alkylols, quaternary compounds, diazo compounds, hydrazides, sulfons, nitro compounds, and ion complexes derived from said water-soluble high-molecular substances by condensation, addition, substitution, oxidation, reduction and other reactions;

Cross-linked products with a substance containing two or more of a reactive functional group such as a diazonium group, an azide group, an isocyanate group, an acid chloride group, an acid anhydride group, an iminocarbonic ester group, an amino group, a carboxyl group, an epoxy group, a hydroxyl group or an aldehyde group; and

Copolymers with a vinyl compound, acrylic acid, methacrylic acid, a dienic compound, maleic anhdydride or the like.

A derivative of these water-soluble high-molecular substances produced by a condensation, addition reaction or a substitution reaction or a product subjected to so-called insolubilization treatment such as partial cross linking may be employed so long as it can be hydrated.

Combination of these substances with a reactive functional group present on or introduced onto at least the surface of a base material for artificial organs by an ionic bond or a covalent bond allows a hydrated layer carried on the base material to form thereby producing durable biocompatible surface without dissolution into water. Any of water-soluble high-molecular substances may be used which most typically include those of cellulose group, maleic anhydride group, acrylamide group, polyethylene oxide group and water-soluble polyamide group. Especially suitable are hydropropyl celluloses, methyl vinyl ether-maleic anhydride copolymers, polyacrylamides, polyethylene glycols, and a water-soluble polyamide (Toray K.K. AQ Nylon P-70) because they are inexpensive, readily available and safe.

There is no particular limit to the average molecular weight of these water-soluble high-molecular

3

EP 0 415 845 A1

substances, but an average molecular weight of around 3-5 millions is preferred because it provides a hydrated layer highly biocompatible, in a moderate thickness and with modest swelling when hydrated.

The reactive functional group present at least on the surface of the base material for artificial organs may be any of those which are capable of reacting with the water-soluble high-molecular substance to fix it by bonding or bridging which are preferably a diazonium group, an azide group, an isocyanate group, an acid chloride group, an iminocarbonic ester group, an amino group, a carboxyl group, an epoxy group, a hydroxyl group and an aldehyde group. Isocyanate, amino, aldehyde and epoxy groups are preferable.

When a base material for artificial organs inherently contains a reactive functional group as in polyurethanes or polyamides, it can directly be combined with a water-soluble high-molecular substance without need of introducing a reactive functional group. However, when a base material for artificial organs containing no reactive functional group is used, it is treated, as described above, with a reactive functional group-containing substance to provide a base material wherein the reactive functional group is present, on which a water-soluble high-molecular substance is then superposed by an ionic bond or a covalent bond. Form of the bond may be ionic or covalent, but the covalent bond is preferable in view of its durability.

As the reactive functional group-containing substance are mentioned polyisocyanates such as ethylene diisocyanate, hexamethylene diisocyanate, xylene diisocyanate, toluene diisocyanate, diphenylmethane diisocyanate, naphthalene diisocyanate, phenylene diisocyanate, cyclohexylene diisocyanate, triphenylmethane triisocyanate and toluene triisocyanate and adducts or prepolymers of these polyisocyanates and polyols. In addition are mentioned low molecular polyamines including ethylenediamine, trimethylenediamine, 1,2-diaminopropane, tetramethylenediamine, 1,3-diaminobutane, 2,3-diaminobutane, pentamethylenediamine, 2,4-diaminopentane, hexamethylenediamine, octamethylenediamine, nonamethylenediamine, decamethylenediamine, undecamethylenediamine, dodecamethylenediamine, tridecamethylenediamine, octadecamethylenediamine, N,N-dimethylethylenediamine, N,N-diethyltrimethylenediamine, N,N-dimethyltrimethylenediamine, N,N-dibutyltrimethylenediamine, N,N,N'-triethylethylenediamine, N-methyltrimethylenediamine, N,N-dimethyl-p-phenylenediamine, N,N-dimethylhex-amethylenediamine, diethylenetriamine, triethylenetetramine, tetraethylenepentamine, heptaethyleneoc-tamine, nonaethylenedecamine, 1,3-bis(2'-aminoethylamino)propane, bis(3'-aminopropyl)amine, 1,3-bis(3'-aminopropylamino)propane, 1,2,3-triaminopropane, tris(2-aminoethyl)amine, tetra(aminomethyl)methane, methyliminobispropylamine, methyliminobisethylamine, ethyliminobisethylamine, N-aminopropyl-2-morpholine, N-aminopropyl-2-pipecoline, N-(2-hydroxyethyl)trimethylenediamine, xylenediamine, phenylenediamine, piperazine, N-methylpiperazine, N-(2-aminoethyl)ethanolamine, N-aminoethylpiperazine, N,N,N',N'-tetramethylethylenediamine and N,N,N'N'-tetramethyltetramethylenediamine.

Also mentioned are high molecular polyamines such as

[I] polyalkylenepolyamines prepared from an amine and an alkylene dihalide or epichlorohydrine (Encyclopedia of Polymer Science and Technology, vol. 10, page 616),

[II] alkylenimine polymers produced by ring-opening polymerization of an alkylenimine such as ethylenimine or propylenimine (Encyclopedia of Polymer Science and Technology, vol. 1, page 734) and

[III] other polyamines including polyvinylamine and polylysine.

Furthermore, there are mentioned

polyaldehydes such as glutaraldehyde, terephthalaldehyde, isophthalaldehyde, dialdehyde, starch, glyoxal, malonaldehyde, succinaldehyde, adipaldehyde, pimelindialdehyde, suberindialdehyde, maleinaldehyde and 2-pentene-1,5-dialdehyde, and

polyepoxides such as ethylene glycol diglycidyl ether, polyethylene glycol diglycidyl ether, propylene glycol diglycidyl ether, polypropylene diglycidyl ether, hexanediol diglycidyl ether and trimethylolpropane triglycidyl ether.

Particularly among them, adduct of 4,4'-diphenylmethane diisocyanate or trilene diisocyanate with trimethylolpropane, adduct of hexamethylene diisocyanate with trimethylolpropane or trimer thereof and diethylenetriamine are most preferred.

There is no particular limitation for the nature of the base material for artificial organs used in the invention. A variety of organic high-molecular base materials for artificial organs such as, for example, polyamide, polyester, polyvinyl chloride, polystyrene, polyacrylic ester; polymethacrylic ester, polyacrilonitrile; polyacrylamide, polyacrylic acid, polymethacrylic acid, polyethylene, polypropylene polyvinyl alcohol, polymaleic anhydride, polyethylenimine, polyurethane, polyvinyl acetate, silicone resin, various latexes and various copolymers and blends thereof as well as a variety of inorganic or metallic base materials for artificial organs including glass, ceramics and stainless steel may be employed. No particular limitation being set in the invention for nature of the base material for artificial organs, the implant materials of the invention can be utilized in a very wide range as described above, for example, for artificial breast, tissue expander, adhesion-preventive membrane, artificial root of a tooth, artificial bone and artificial joint.

4

These base materials for artificial organs may contain various additives as required.

Formation of the coat layer according to the invention upon such base materials for artificial organs may be effected by procedures as described below.

When a conventional base material for artificial organs containing no reactive functional group is used, a substrate layer is first formed.

Formation of the substrate layer can be made merely by dipping the base material for artificial organs in a solution containing a reactive functional group-containing compound as mentioned above followed by drying.

Solvents, for example, ketones such as methyl ethyl ketone, methyl isobutyl ketone and cyclohexanone, esters such as butyl acetate, ethyl acetate, carbitol acetate and butylcarbitol acetate, ethers such as methylcellosolve, ethylcellosolve and tetrahydrofuran, aromatics such as toluene and xylene, halogenated alkyls such as dichloroethane and alcohols may be used as the solvent in the above formation.

It is to be noted that the solvent is preferably those which will dissolve or swell the resin base material for artificial organs (in some cases, the preformed resin coated layer on the surface of said base material for artificial organs). This allows for increased adhesion strength and durability of the coat layer.

Especially preferable solvents of such nature include methyl ethyl ketone, cyclohexanone, tetrahydrofuran, xylene and methyl alcohol.

In addition to the dipping treatment, such treatments as brushing and spinner may also be applied.

Subsequent drying step is for evapotranspiration of the solvent which may be carried out usually at room temperature to about 80°C for 5 min. to about 48 hours.

When the surface of a base material for artificial organs is in advance coated with an adhesive layer, the coating may be carried out in a conventional way.

Then, the base material for artificial organs with a substrate layer formed (in some cases, without a substrate layer formed) is treated with a solution containing a water-soluble high-molecular substance of the invention.

The solvent used in the above treatment is those which are not reactive with the reactive functional group present in the base material for artificial organs or the adhesive layer thereon, that is, are unreactive with the isocyanate group and amino group, and preferably includes methyl ethyl ketone, THF and acetone. Those which will dissolve or swell the base material for artificial organs are especially preferred.

Concentration of the solution is preferably about 0.1-15%, more particularly about 0.5-10%.

The above treatment is run in the same way as above, usually by dipping. Various other coating procedures may also be employed.

The treatment temperature is in the range from room temperature to 80°C. The treatment time may roughly be in the range from 1 sec. to 48 hours. Sequentially, the drying treatment is in the range from room temperature to 80°C and its treatment time may roughly be in the range from 5 min. to 48 hours.

[Action and effect of the invention]

The implant materials of the invention are provided with a coat layer comprising a water-soluble high-molecular substance or a derivative thereof formed on the surface of a base material for artificial organs via a reactive functional group, and consequently, even when implanted in vivo for a long period of time they can be nearly completely free from inflammation reactions and adhesion with a thinner capsule layer formed thereon and consequently less bleeding and pain associated.

It is also feasible that, depending upon the region for use as the implant materials, the base material for artificial organs be selected to meet physico-engineering requirements so that applicability of the implant materials extends over a very wide range. Thus, as described above, the materials are applicable at a variety of regions from artificial breast, tissue expander and adhesion-preventive membrane which require flexibility to artificial root of a tooth, artificial bone and artificial joint which require hardness.

The invention will be described with reference to examples below.

[Example 1]

A 10% w/v ethanol solution of a methyl vinyl ether-maleic anhydride (GANTRETZ AN-169; MW = 1,500,000, manufactured by GAF) was allowed to react with stirring at the refluxing temperature (73°C) for 24 hours. The solution was subjected to poor solvent replacement by hexane. The solids thus produced was dried, pulverized and further dried in vacuo to obtain a powdery methyl vinyl ether/monoethyl maleate copolymer (hereinbelow called PVM/ME for short). Degree of esterification for the PVM/ME obtained was 47-50%.

[Example 2]

A sheet of polyvinyl chloride (called PVC hereinbelow) 20 x 20 mm in size and 0.4 mm in thickness was dipped in a 1% w/v methyl ethyl ketone (called MEK hereinbelow) of methylenediphenyl 4,4′-diisocyanate (called MDI (hereinbelow) for one min. followed by drying in an oven at 60°C for 30 min.

The sheet was then immersed in a 1% w/v tetrahydrofuran (called THF hereinbelow) of the PVM/ME obtained in Example 1 for one sec. followed by dring in an oven at 60°C for 24 hours.

The resulting sheet was washed in an aqueous solution of 0.01% w/v $NaHCO_3$ and 0.9% w/v NaCl in tap water at 45°C for 2 hours and then washed with tap water and distilled water, dried and then sterilized with ethylene oxide gas (called EOG hereinbelow) to give Sample A.

[Example 3]

A sheet of a polystyrene resin was dipped in a 2% w/v acetone solution of MDI and then dried in an oven at 60°C for 30 min.

The sheet was then dipped in a 1% w/v solution of the PVM/ME obtained in Example 1 in a mixed solvent of THF/acetone (THF:acetone = 1:2 w/v) for one sec. followed by drying in an oven at 60°C for 20 hours.

The resulting sheet was subsequently washed and sterilized in the same way as in Example 2 to give Sample B.

[Example 4]

A sheet of polycarbonate (Mitsubishi IUPILON was treated in the same way as in Example 3 to give Sample C.

[Example 5]

A circular sheet of PVC 20 mm in diameter and 0.4 mm in thickness having the same quality as the one used in Example 2 was dipped in a 1% w/v THF solution of MDI for 5 sec. followed by drying in an oven at 60°C for 30 min.

Then, the sheet was dipped in a 1% w/v THF solution of the PVM/ME obtained in Example 1 for one sec. followed by drying in an oven at 60°C for 20 hours.

The resulting sheet was subsequently washed and sterilized in the same way as in Example 2 to give Sample D.

[Example 6]

A silicone sheet for medical use 20 mm in diameter and 0.4 mm in thickness (medical use of Dow Corning SILASTIC 500-5, a sheet for transplant material) was dipped in a 1% w/v THF solution of MDI for 10 sec. and then dried in an oven at 60°C for 30 min.

Then, the sheet was dipped in a 1% w/v THF solution of the PVM/ME obtained in Example 1 for 5 sec. followed by drying in an oven at 60°C for 20 hours.

The resulting sheet was subsequently washed and sterilized in the same way as in Example 2 to give Sample E.

[Comparative Example 1]

A PVC sheet of the same quality and shape as the one used in Example 2 was sterilized with EOG to give Sample F.

[Comparative Example 2]

A medical polyurethane elastomer (M.D. Kasei Pellethane 2363-65D medical grade) was formed into a sheet 20 x 20 mm in size and 0.5 mm in thickness which was then sterilized to give Sample G.

[Comparative Example 3]

A sheet of polypropylene (Mitsubishi Yuka MA6) 20 x 20 mm in size and 0.5 mm in thickness was sterilized with EOG to give Sample H.

[Comparative Example 4]

A medical silicone.of Dow Corning (SILASTIC MDX 4-4210, medical grade) was cured at 80°C for 30 min. to prepare a sheet 20 x 20 mm in size and 0.5 mm in thickness which was then sterilized with EOG to give Sample I. (Comparative Example 5)

A hydroxy apatite sintered product 15 mm in diameter and 1 mm in thickness was formed by calcination at 850°C for 2 hours, subsequent granulation and sintering at 1300°C for 2 hours and sterilized with EOG to give Sample J.

[Comparative Example 6]

A sheet of PVC 20 mm in diameter and 0.4 mm in thickness of the same quality and shape as the one used in Example 5 was dipped in THF for 5 sec. followed by drying in an oven at 60°C for 20 hours. The resulting sheet was washed and sterilized in the same way as in Example 2 to give Sample K.

[Comparative Example 7]

A silicone sheet of the same quality and shape as the one used in Example 6 was sterilized with EOG to give Sample L.

[Test Example 1] (Intraperitoneal implantation test: Rat)

Sample A prepared in Example 2 was intraperitoneally implanted in the rat and evaluated for adhesion and inflammation. Wistar-KY female rats weighing about 200 g were used. The animal was anesthesized by itravenous administration of pentobarbital at a dose of 40 mg/kg body weight. The abdomen was wetted with isodine for surgical operation and shaved by a shaving razor. The skin was sterilized with hibitane-alcohol and then longitudinally incised in the midline (approximately 2 cm in length) together with the peritoneum. The sample was inserted inside the peritoneum. The opening was closed in knots with a polyamide clip suture. As a control, the sample composed only of the base material for artificial organs of the same size (Comparative Example 1) was implanted one by one in another group of rats. On day 28 and day 56, one rat of each group was killed, and the implanted material was removed for gross observation. The material was then fixed with a 10% neutrally buffered formalin and histopathologically examined. Thus, the implanted material after completion of the fixing was cut in strip approximately 0.5 cm in width, washed with water, then thoroughly soaked with ethanol, and then with toluene which was replaced by a paraffin solution (60°C). The paraffin embedded material was cooled to prepare paraffin blocks. The paraffin block was sliced with a rotary microtome manufactured by Yamato in slice 4 um in thickness. The sliced piece was placed on a slide glass, HE stained and histopathologically observed.

The results are shown in Table 1 below.

Table 1

| (Gross and Histopathological Findings in the Rat Intraperitoneal Implantation Test) | | | | |
|---|---|---|---|---|
| | After 28 days | | After 56 days | |
| Sample | Gross observation | Histopathological observation | Gross observation | Histopathological observation |
| Coated PVC (Example 2) | No Adhesion | Thin capsule formation without inflammatory cell filtration | No adhesion | Thin capsule formation only |
| Uncoated PVC (Comparative Example 1) | Adhered with adipose tissue | Sample peripherally surrounded by monocytes with capsule formed externally and transferred to adipose tissue | Adhered with adipose tissue | Sample peripherally surrounded by monocytes with capsule formed externally and transferred to adipose tissue |

There is observed in the uncoated PVC apparent adhesion with the adipose tissue. Histologically, monocytic reactions are remarkable both on days 28 and 56. Thus the uncoated PVC is considered to be a highly foreign material and have low biocompatibility. On the contrary, in the coated PVC (Example 2) according to the invention there is observed that adhesion is completely prevented and no inflammated cell reaction occurs. The observations suggest that the material is less foreign and highly biocompatible.

[Test Example 2] (Subcutaneous implantation test: Rats)

The sample prepared in Example 2 was subcutaneously implanted in rats on the back and evaluated for biocompatibility. Wistar-KY female rats weighing approximately 200 g were used. The animal was anesthesized by intravenous administration of pentobarbital at a dose of 40 mg/kg body weight. The back was wetted with isodine for surgical operation and shaved by a shaving razor. The skin of the back was sterilized with hibitane-alcohol and then longitudinally incised (approximately 2 cm in length). Scissors were put in through the incision to form two subcutaneous pockets on the right and left sides. One piece of the sample was inserted into the right and left pockets, respectively. The samples used included, in addition to the coated PVC prepared in Example 2, untreated PVC (Comparative Example 1), medical polyurethane (Comparative Example 2), polypropylene (Comparative Example 3), medical silicone (Comparative Example 4) and a hydroxyapatite sintered product (Comparative Example 5) as controls. Eight weeks after the implantation the animals were autopsied. After gross observation the test piece was removed together with the surrounding skin tissue and fixed with formalin. After the fixation the skin was cut, the subcutaneously implanted test piece carefully extracted, and the peripherally formed tissue histopathologically examined in the same way as in Test Example 1.

The results were as follows:

In both cases of the medical polyurethane and the polypropylene the test piece pierced the skin at the time when 7 weeks elapsed to expose a part of the test piece. There was observed no remarkable change in the other cases. Gross findings and pathological findings in the region surrounding the test piece are shown in Table 2.

Table 2

| Gross and Pathological Findings in the Subcutaneous Implantation Test in Rats (After 8 weeks) | | | |
|---|---|---|---|
| Sample | Gross finding | Pathological finding | Capsule thickness ($\mu$m) |
| Example 2 | No remarkable change | Thin capsule formation | 160.0 |
| Comparative Example 1 | No remarkable change | Monocyte infiltration + capsule formation | 186.7 |
| Comparative Example 2 | Test piece exposed | (Region remaining under the skin) Capsule formation + cytometaplasia | 191.0 |
| Comparative Example 3 | Test piece exposed | (Region remaining under the skin) Capsule formation + cytometaplasia | 200.0 |
| Comparative Example 4 | No remarkable change | Capsule formation | 196.0 |
| Comparative Example 5 | No remarkable change | Capsule formation | 198.0 |

The above results indicated that the material according to the invention wherein PVC was coated with PVM/ME not only masked intrinsic bioincompatibility of the base material for artificial organs but also was superior in formation of the capsule layer to prior-art materials.


[Test Example 3] (Intraperitoneal Implantation Test: Mice)

Effects of the implant materials according to the invention were evaluated in intraperitoneal implantation in mice run in the same way as in Example 1.

The samples used are those obtained in Examples 5 and 6 and Comparative Examples 6 and 7.

On days 14, 28 and 56 after implantation two mice of each group were killed and subjected to gross and histopathological examinations of the test piece and the peripheral tissue. The results are shown in Table 3 below.

Table 3

| Gross (upper column) and Pathological (lower column) Findings in the Intraperitoneal Implantation Test in Mice | | | |
|---|---|---|---|
| Sample | After 14 days | After 28 days | After 56 days |
| Example 5 (Coated PVC) | No adhesion / Monocytes attached | No adhesion / Mesothelial cells lively attached or thin capsule formed | No adhesion / Mesothelial cells lively attached or thin capsule formed |
| Comparative Example 6 (Uncoated PVC) | Viscera adhered / Monocytes attached + capsule formed (bilayered capsule) | Viscera adhered / Bilayred capsule | No adhesion / Bilayered capsule, partial calcification |
| Example 6 (Coated silicone) | No adhesion / Monocytes attached | No adhesion / Thin capsule formed | Organ adhered / Thin capsule formed or monocytes attached |
| Comparative Example 7 (Uncoated silicone) | No adhesion / Capsule formed | Viscera adhered, big hepatoma / [Peritonitis induced] capsule formed | Big hepatoma, retention of red ascites / [Peritonitis induced] capsule formed formed or monocytes lively attched |

As seen from Table 3, coating of the PVC base material for artificial organs with the water-soluble high-molecular substance produced similar effects to those observed in Test Example 1 (rats) such as prevention of adhesion, reduced inflammation and reduction in capsule thickness. These effects are often produced in cases where mesothelial cells which are epitherial cells in the abdominal cavity are lively attached via a thin substrate (bisilar membrane) to a layer of the water-soluble high-molecular substance.

As also seen from Table 3, coating of the medical silicone base material for artificial organs with the water-soluble high-molecular substance of the invention almost inhibited induction of peritonitis, which was induced by the uncoated silicone, and was accompanied only by formation of thin capsule.

[Test Example 4] (Toxicity Test: Mice)

The coated PVC sheet prepared in Example 2 was hydrated by immersion in a physiological saline solution and thoroughly washed with distilled water to remove the salt. Subsequently, the coating layer on the surface of the sheet was forcibly scraped off with a spatula and suspended in a physiological saline solution. The suspension, adjusted to a concentration of 247 µg/ml, was intravenously administered to mice from the tail at doses of 32.4 and 67.5 ml/kg, respectively. No death occurred in every group.

Histopathologically, all of the administered fine pieces remained in the pulmonary capillary blood vessels as emboli. During weeks 1 to 4 after the administration infiltration of mononuclear leucocytes was observed in the embolic region. The infiltration disappeared in and subsequent to week 6, when only a very small number of fibroblasts were scattered around and between the emboli. Re-facilitation began to occur in the embolic region in and subsequent to week 4, and coating began on the surface of the fine pieces via a thin substrate with hemendothelial cells. Thereafter, for the following 65 weeks, it was observed that the fine pieces were covered with a layer of the endothelial cells the internal crevices of which contained a small number of fibroblasts but neither inflammation nor malignant alteration observed at all.

Whereas a maleic anhydride high-molecular substance was used as the water-soluble high-molecular substance in the examples, similar results were produced with cellulose high-molecular substances, ethylene oxide high-molecular substances and water-soluble polyamide high-molecular substances, too. The invention shall not be limited to the examples.

## Claims

(1) An implant material for long-term implantation comprising a base material for artificial organs coated, at least on its surface to be in contact with biotissues, with a water-soluble high-molecular substance or a derivative thereof wherein said water-soluble high-molecular substance or a derivative thereof is combined with at least a reactive functional group present on the surface of the base material for artificial organs by an ionic bond or a covalent bond.

(2) An implant material for long-term implantation according to claim 1 wherein the reactive functional group is introduced by treating the surface of a base material for artificial organs with a solution of a compound having the reactive functional group.

(3) An implant material for long-term implantation according to claim 1 or 2 wherein the reactive functional group is an aldehyde group, an epoxy group, an isocyanate group or an amino group.

(4) An implant material for long-term implantation according to claim 1, 2 or 3 wherein the water-soluble high-molecular substance is a cellulose high-molecular substance, a maleic anhydride high-molecular substance, an acrylamide high-molecular substance, a polyethylene oxide high-molecular substance or a water-soluble polyamide.

(5) An implant material for long-term implantation according to claim 4 wherein the cellulose high-molecular substance is hydroxypropylcellulose.

(6) An implant material for long-term implantation according to claim 4 wherein the maleic anhydride high-molecular substance is a methyl vinyl ether-maleic anhydride copolymer.

(7) An implant material for long-term implantation according to claim 4 wherein the acrylamide high-molecular substance is a polyacrylamide.

(8) An implant material for long-term implantation according to claim 4 wherein the polyethylene oxide high-molecular substance is a polyethylene glycol.

(9) A process for preparing an implant material for long-term implantation which comprises treating a base material for artificial organs with a solution of a reactive functional group-containing compound to form a substrate layer of said base material for artificial organs having the reactive functional group at least on the surface of said base material and then treating the substrate layer with a water-soluble high-molecular substance or a derivative thereof to combine the reactive functional group with the water-soluble high-molecular substance by an ionic bond or a covalent bond thereby forming a coat layer of the water-soluble high-molecular substance on said substrate layer.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 166 998 (TERUMO K.K.) <br> * Whole document * | 1-9 | A 61 L 27/00 |
| X | EP-A-0 106 004 (INTERNATIONAL SILICONE CORP.) <br> * Page 2, lines 20-35; page 7, lines 25-34; page 8, lines 27-35 * | 1-3,9 | |
| X | EP-A-0 028 122 (UNITIKA LTD) <br> * Page 9, lines 13-23; page 12, lines 1-23; page 4, lines 16-21; page 15, lines 12-16; page 16, lines 8-18 * | 1 | |
| A | WO-A-8 602 087 (YTKEMISKA INSTITUTET) <br> * Claims * | 1 | |
| A | EP-A-0 093 094 (ASTRA MEDITEC AB) <br> * Page 3, lines 21-26; page 4, lines 13-20 * | 1 | |
| A | BIOMATERIALS, vol. 10, no. 1, January 1989, pages 3-10, Butterworth <br> & Co. (Publishers) Ltd, Guildford, Surrey, GB; P.H. CORK-HILL et al.: "Synthetic hydrogels. VI. Hydrogel composites as wound dressings and implant materials" <br> * Page 8 * | 1 | TECHNICAL FIELDS SEARCHED (Int. Cl.5) <br><br> A 61 L |

The present search report has been drawn up for all claims

| Place of search | Date of completion of search | Examiner |
|---|---|---|
| The Hague | 07 November 90 | ESPINOSA Y CARRETERO |